# EUROPEAN PATENT APPLICATION

(11) **EP 1 795 563 A1**
(43) Date of publication of application: **13.06.2007**
(21) Application number: 06256107.1
(22) Date of filing: 29.11.2006
(51) Int. Cl.: C09D 4/00

(54) **Amphiphilic polymeric coating**

(30) Priority: 30.11.2005 US 290692
(71) Applicant: Cordis Corporation, Miami Lakes, Florida 33014 (US)
(72) Inventor: Narayanan, Pallassana, Belle Mead, NJ 08502 (US); Zhao, Jonathon Z, Belle Mead, NJ 08502 (US)
(74) Representative: Belcher, Simon James

(57) **Abstract**

An amphiphilic coating material for applying on at least a portion of one surface of an article comprises a polymer having a hydrophobic backbone derived from a vinyl moiety and hydrophilic pendent chains derived from a polyethylene oxide moiety. The hydrophobic backbone derived from a vinyl moiety adheres to at least one surface of an article. The hydrophilic pendent chains derived from a polyethylene oxide moiety extend from the hydrophobic backbone and form a three-dimensional network.

## Description

The present invention relates to an amphiphilic coating material for application to at least a portion of one surface of an article. The present invention also relates to an article having the amphiphilic coating.

Most medical devices are made from metals, ceramics, or polymeric materials. However, these materials are hydrophobic, non-conformal, and non-slippery, and thereby may cause injury or inflammation of mucous membranes during use or operation. Thus, the issue of biocompatibility is a critical concern for manufacturers of medical devices, particularly medical implants. In order to function properly and safely, medical devices are usually coated with one or more layers of biocompatible materials. The coatings on these medical devices may, in some instances, be used to deliver therapeutic and pharmaceutical agents.

Since medical devices, particularly implantable medical devices, are intended for prolonged use and directly interface with body tissues, body fluids, electrolytes, proteins, enzymes, lipids, and other biological molecules, the coating materials for medical devices must meet stringent biological and physical requirements. These requirements, as a minimum, include the following criteria: (1) the coatings must be lubricious when in contact with body fluids, but non-slippery at dry conditions; (2) the coatings must be flexible and elastic, so they conform to the biological structure without inducing detrimental stress; (3) the coatings must be hydrophilic and haemocompatible; (4) the coatings must be chemically inert to body tissue and body fluids; and (5) the coatings must be mechanically durable and not crack when formed on medical devices. If the coatings are impregnated with pharmaceutical or therapeutic agents, it is typically required that the coatings and the formation thereof are compatible with the pharmaceutical or therapeutic agents. If the coatings are used as coatings and the underlying basecoats are impregnated with pharmaceutical or therapeutic agents, it is further required that the coating and the formation thereof must be compatible with the basecoat and the pharmaceutical or therapeutic agents impregnated therein; and the coating must allow the pharmaceutical or therapeutic agents to permeate through it. It is also desirable that the coating functions as a physical barrier, a chemical barrier, or a combination thereof to control the elution of the pharmaceutical or therapeutic agents in the underlying basecoat.

A typical coating composition of the prior art comprises a supporting polymer and a hydrophilic polymer, in which the supporting polymer contains functional moieties capable of undergoing crosslinking reactions and the hydrophilic polymer is associated with the supporting polymer, for example as disclosed in US-6238799). However, the preparation of the prior art coating composition employs chemical crosslinking reactions and a high temperature curing process, which are not compatible with a drug-containing coating.

It is also known to use a coating composition formed by the gas phase or plasma polymerization of a gas comprising monomers of polyethylene glycol vinyl ether compounds, for example as disclosed in US-A-2003/0113477. However, the polymer prepared through the plasma process has a poorly defined molecular weight and a large polydispersity. The plasma laid polymers of low molecular weight have limited mechanical durability. Further, plasma treatment can penetrate through the underlying basecoat and damage the drug content therein. Another problem with the prior art approach is that the free radicals or other high energy species generated in the plasma process may persist in the coating and cause drug content loss in the basecoat over time.

Thus, there remains a need for a coating material that can be applied on at least a portion of one surface of a medical device and that can satisfy all the requirements described above.

Accordingly, the present invention provides an amphiphilic coating material for applying on at least a portion of one surface of an article. By "amphiphilic", it is meant having the property of hydrophobicity and hydrophilicity simultaneously. The amphiphilic coating material comprises a polymer having a hydrophobic backbone derived from a vinyl moiety and hydrophilic pendent chains derived from a polyethylene oxide moiety.

Preferably, the polymer contains a monomer unit comprising one of the following two structures: 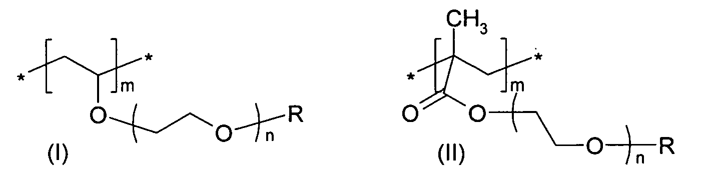 in which R is a hydrogen atom or an alkyl group of 1 to 6 carbon atoms; n is an integer of 2 to 100; and m is an integer of 100 to 5000.

The polymer may also be a copolymer comprising a monomer unit containing one of the following structures: 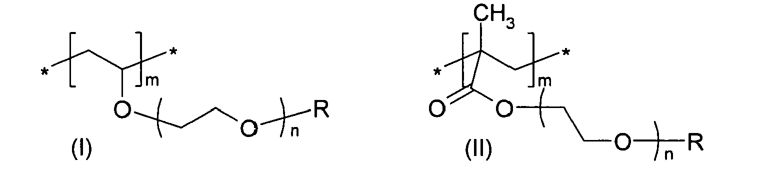 in which R is a hydrogen atom or an alkyl group of 1 to 6 carbon atoms; n is an integer of 2 to 100; and m is an integer of 100 to 5000; and up to 50% mole of one or more methacrylate or acrylate co-monomer units.

The present invention also provides an article having an amphiphilic coating thereon. The amphiphilic coating comprises a polymer having a hydrophobic backbone derived from a vinyl moiety and hydrophilic pendent chains derived from a polyethylene oxide moiety. The hydrophobic backbone derived from a vinyl moiety adheres to at least a portion of one surface of an article. The hydrophilic pendent chains derived from a polyethylene oxide moiety extend from the hydrophobic backbone and form a three-dimensional network.

Embodiments of the invention will now be described by way of example with reference to the accompanying drawings, in which:
FIG. 1 is a pictorial illustration of an article coated with the amphiphilic coating material.
FIG. 2 is a pictorial illustration of an article coated with a basecoat and the amphiphilic coating material.

The present invention provides an amphiphilic coating material for applying on at least a portion of one surface of an article. The amphiphilic coating material comprises a polymer having a hydrophobic backbone derived from a vinyl moiety and hydrophilic pendent chains derived from a polyethylene oxide moiety. By "hydrophobic backbone derived from a vinyl moiety", it is meant the hydrophobic component of the polymer which is formed by polymerization of vinyl groups. By "hydrophilic pendent chains derived from a polyethylene oxide moiety", it is meant the hydrophilic component of the polymer which comprises polyethylene oxide groups.

Preferably, the polymer contains a monomer unit comprising one of the following two structures: 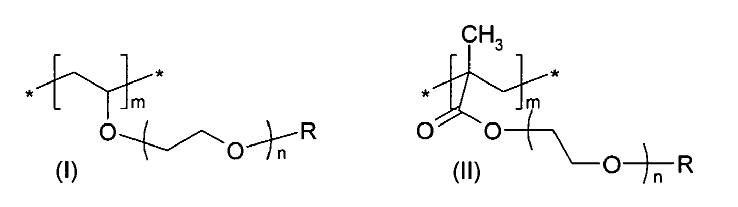 in which R is a hydrogen atom or an alkyl group of 1 to 6 carbon atoms; n is an integer of 2 to 100; and m is an integer of 100 to 5000. Preferably, n is an integer of 2 to 10. The alkyl group suitable for the present invention may be straight, branched, or cyclic. Examples of suitable alkyl groups include, but are not limited to: methyl, ethyl, propyl, isopropyl, butyl, isobutyl, *tert*-butyl, *n*-pentyl, cyclopropyl, cyclobutyl, and cyclopentyl. Preferably, the alkyl group is methyl.

The polymer may also be a copolymer comprising a monomer unit containing one of the following structures: 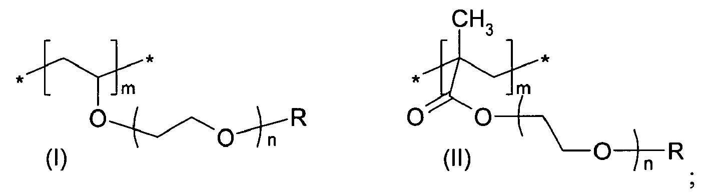 in which R is a hydrogen atom or an alkyl group of 1 to 6 carbon atoms; n is an integer of 2 to 100; and m is an integer of 100 to 5000; and up to 50% mole of one or more methacrylate or acrylate co-monomer units.

When the copolymer comprises a monomer containing a structure of formula (I) or (II) and more than one co-monomers, the more than one co-monomers can be the same or different. In accordance with the present invention, the one or more co-monomers include, but are not limited to: methacrylates and/or acrylates. Preferred methacrylate co-monomers include methyl methacrylate, ethyl methacrylate, alicyclic methacrylate, or acyclic alkyl substituted methacrylates in which the alkyl and the alicyclic can independently have 1 to 12 carbon atoms. The preferred acrylate co-monomers include methyl acrylate, ethyl acrylate, alicyclic acrylate, acyclic alkyl substituted acrylates, acrylic acid, hydroxyethyl acrylate, hydroxypropyl acrylate, hydroxy substituted alicyclic acrylate, and acyclic hydroxyalkyl substituted acrylates, in which the alkyl and the alicyclic independently have 1 to 12 carbon atoms. It is understood to one skilled in the art that suitable co-monomers also include any analogous methacrylates or acrylates of the above-mentioned methacrylate and acrylate co-monomers.

The polymer can consists essentially of a repeating monomer unit of formula (I) or (II): 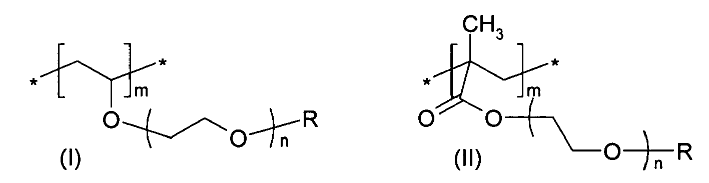 in which R is a hydrogen atom or an alkyl group of 1 to 6 carbon atoms; n is an integer of 2 to 100; and m is an integer of 100 to 5000. Preferably, n is an integer of 2 to 10.

The polymer can be a copolymer comprising the following repeating unit: 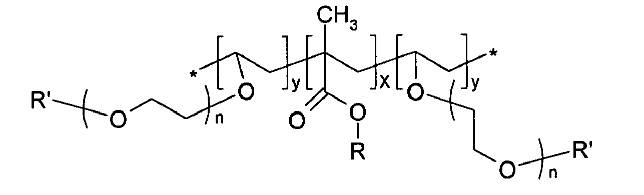 in which R and R' are the same or different, and are independently a hydrogen atom or an alkyl group of 1 to 6 carbons; n is an integer of 2 to 100; and x and y are the same or different, and are independently an integer of 100 to 5000.

The polymer can be a copolymer comprising the following repeating unit: 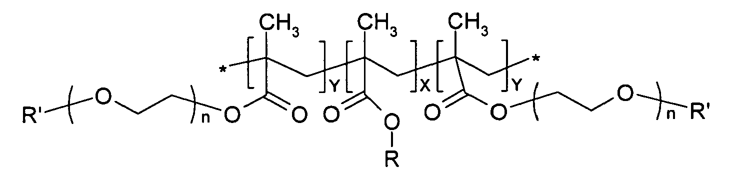 in which R and R' are the same or different, and are independently a hydrogen atom or an alkyl group of 1 to 6 carbons; n is an integer of 2 to 100; and X and Y are the same or different, and are independently an integer of 100 to 5000.

The amphiphilic coating material may be applied on at least a portion of one surface of an article. The polymer can be applied to all exposed surfaces of an article. The thickness of the amphiphilic coating may vary depending on the process used in forming the coating as well as the intended use of the article. Typically, and for a medical device, the coating is applied to a thickness from about 1 to about 500 nm, with a thickness from about 2 to about 100 nm being more typical.

It is preferable that the polymer has a tunable polymer molecular weight ranging from about 5 kDa to about 500 kDa to enable the formation of a polymer with desirable mechanical durability and adequate adhesiveness. Since the mechanical durability of a coating improves upon increasing polymer molecular weight, it is especially preferable that the polymer has a high polymer molecular weight of 10 kDa to 500 kDa for use in coatings for certain medical devices (e.g., stents) which require expansion and deployment *in vivo.* Co-monomers can also be added to prepare copolymer materials with improved mechanical durability and adhesiveness.

The amphiphilic coating of the present invention may additionally include cosolvents and/or other additives to facilitate high quality film formation, such as plasticizers, antifoaming agents, anticrater agents, and coalescing solvents. Other suitable additives to the amphiphilic coating material include, but are not limited to: bioactive agents, antimicrobial agents, antithrombogenic agents, antibiotics, pigments, radiopacifiers and ion conductors. Details concerning the selection and amounts of such ingredients are known to those skilled in the art.

When applied on at least one surface of an article, the hydrophobic backbone derived from a vinyl moiety forms a non-swellable base layer and adheres firmly to the underlying surface, while the hydrophilic pendent chains derived from a polyethylene oxide moiety hydrate and swell under physiological conditions and form a lubricious and haemocompatible surface. The low-friction and haemocompatibility of the hydrophilic pendent chains provide excellent anti-thrombotic properties that potentially reduce subacute thrombosis (SAT). Further, the hydrophobic backbone derived from a vinyl moiety has a predefined molecular weight with a narrow range of distribution which improves the mechanical durability of the polymer, while the hydrophilic pendent chains derived from a polyethylene oxide moiety is adjustable to various lengths to obtain the desirable elasticity of the polymer. Thus, the polymer is robust, i.e., mechanically durable, and flexible, i.e., elastic. The robustness and flexibility of the coating significantly improve flaking, peeling, and other defects commonly seen in many current coatings on medical devices, particularly the coatings on stents. Accordingly, the present invention provides an improved biocompatible coating, which has both inert hydrophilic surfaces to be in contact with body tissue of a mammal, for example, a human, sufficiently lubricious to reduce restenosis, or thrombosis, or other undesirable reactions, and a hydrophobic backbone to firmly adhere to the underlying surface sufficiently durable to resist cracking when formed on an article, for example, a medical device.

The amphiphilic coating may also be applied to control the elution of a therapeutic dosage of a pharmaceutical agent from a medical device base coating, for example, a stent base coating. The basecoat generally comprises a matrix of one or more drugs, agents, and/or compounds and a biocompatible material such as a polymer. The control over elution results from either a physical barrier, or a chemical barrier, or a combination thereof. The elution is controlled by varying the thickness of the coating, thereby changing the diffusion path length for the drugs, agents, and/or compounds to diffuse out of the basecoat matrix. Essentially, the drugs, agents and/or compounds in the basecoat matrix diffuse through the interstitial spaces in the coating. Accordingly, the thicker the coating, the longer the diffusion path, and conversely, the thinner the coating, the shorter the diffusion path. It is important to note that both the basecoat and the coating thickness may be limited by the desired overall profile of the article on which they are applied.

The structure of the hydrophobic backbone and the molecular weight of the polymer may be controlled or tuned through employment of various polymerization methods. The preferred polymerization methods of the present invention include group transfer polymerization (GTP), anionic polymerization, and living polymerization. The more preferred polymerization method of the present invention is GTP. GTP is a living polymerization technique which involves a Michael-type addition using a silyl ketene acetal initiator (See, for example, Vamvakaki, M. et al., Polymer, 40, 1999, 5161-5171). Many conventional polymerization methods require chemical crosslinking reactions, high temperature curing processes, and/or plasma treatments, which not only have very limited control over the polymer backbone structure and the molecular weight distribution, but also cause damages to the drug-content in the underlying basecoat. Unlike those conventional polymerization methods, GTP may be used for the synthesis of controlled structure acrylate or methacrylate polymers of narrow molecular weight distribution at ambient temperature. The hydrophilic pendent chains derived from a polyethylene oxide moiety provide desired lubricious properties and haemocompatibility, and the length of those hydrophilic pendent chains can be controlled by using monomers with desirable number of repeating ethylene oxide unit in the polymerization reactions. The preferred monomers for the polymerization are the monomers that contain 2 to 10 repeating ethylene oxide units.

A general polymerization process is shown in Scheme 1 as below: 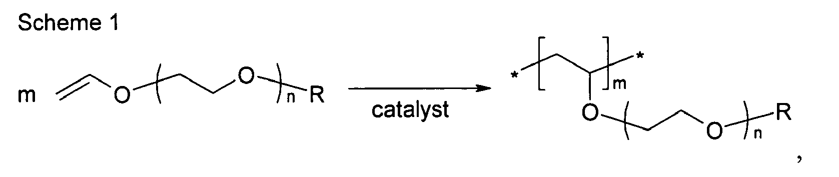 in which R is a hydrogen atom or an alkyl group having 1 to 8 carbon atoms, m is an integer of 100 to 5000, and n is an integer of 2 to 100. Catalysts suitable for the above polymerization process are known to one skilled in the art. Examples of the catalysts include, but are not limited to: 1-methoxy-1-trimethylsiloxy-2-methyl-1-propene (MTS), *n-*tetrabutylammonium bibenzoate (TBABB), and other polymerization initiators.

A general co-polymerization process is shown in Scheme 2 as below: 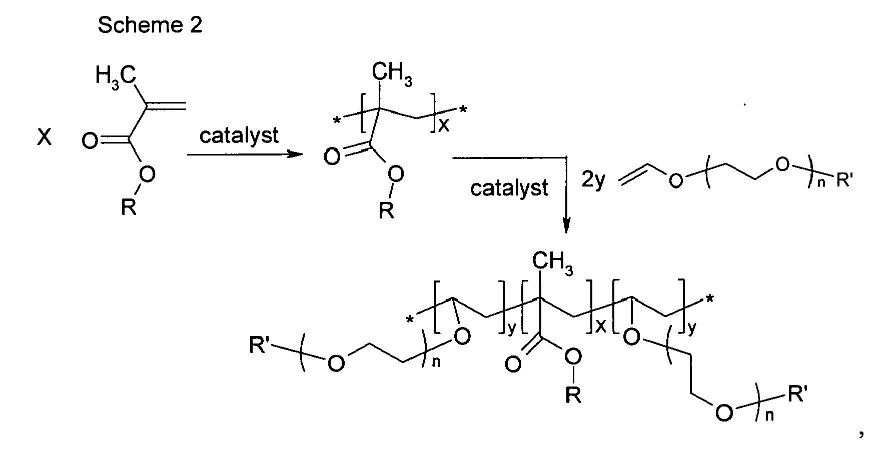 in which R and R' are independently a hydrogen atom or an alkyl group having 1 to 8 carbon atoms, x and y are independently an integer of 100 to 5000, and n is an integer of 2 to 100. Catalysts suitable for the above polymerization process are known to one skilled in the art. Examples of the catalysts include, but are not limited to: 1-methoxy-1-trimethylsiloxy-2-methyl-1-propene (MTS), *n*-tetrabutylammonium bibenzoate (TBABB), and other polymerization initiators.

Another general co-polymerization process is shown in Scheme 3 as below: 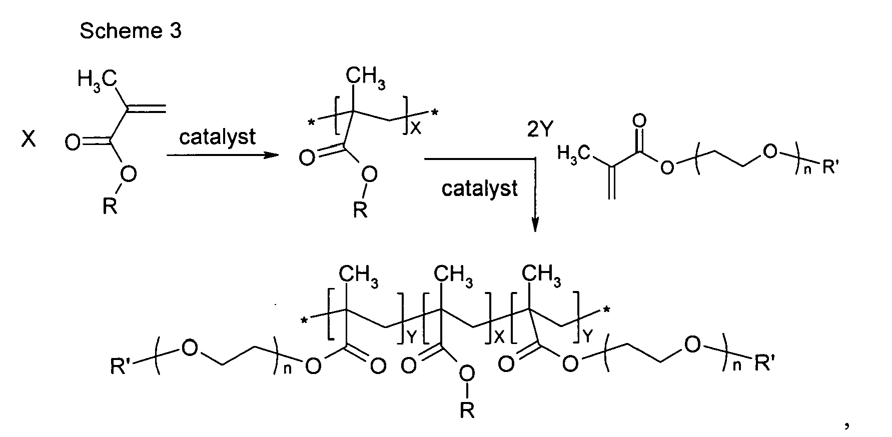 in which R and R' are independently a hydrogen atom or an alkyl group having 1 to 8 carbon atoms, X and Y are independently an integer of 100 to 5000, and n is an integer of 2 to 100. Catalysts suitable for the above polymerization process are known to one skilled in the art. Examples of the catalysts include, but are not limited to: 1-methoxy-1-trimethylsiloxy-2-methyl-1-propene (MTS), *n*-tetrabutylammonium bibenzoate (TBABB), and other polymerization initiators.

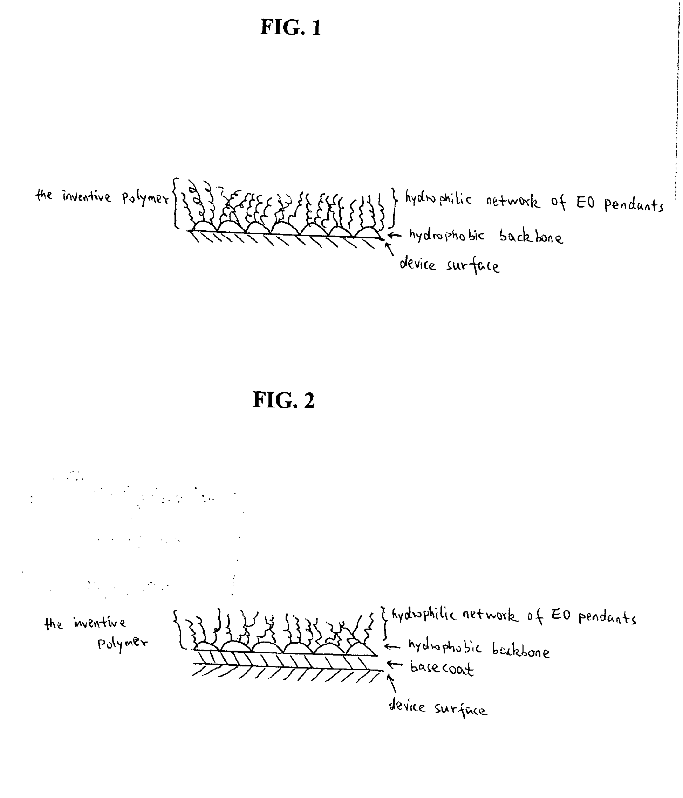

The present invention also provides an article having an amphiphilic coating thereon. The amphiphilic coating comprises a polymer having a hydrophobic backbone derived from a vinyl moiety and hydrophilic pendent chains derived from a polyethylene oxide moiety. The hydrophobic backbone derived from a vinyl moiety is on top of, and in direct contact with, at least one surface of the article. That is, the hydrophobic backbone derived from a vinyl moiety adheres firmly on at least a portion of one surface of the article. The at least a portion of one surface of the article may be a surface of a polymeric coat, a plastic substance, ceramic, steel, or other alloy metals. The hydrophilic pendent chains derived from a polyethylene oxide moiety extend from the hydrophobic backbone and form a three-dimensional network. FIG. 1 is a pictorial illustration of an article having the amphiphilic coating thereon, in which the hydrophobic backbone of the polymer adheres directly to the surface of the article. FIG. 2 is a pictorial illustration of an article having the amphiphilic coating thereon, in which the hydrophobic backbone of the polymer adheres to the underlying basecoat. In these drawings, reference number 10 denotes the polymer, 12 denotes the device surface, 14 denotes the hydrophobic backbone, 16 denotes the hydrophilic network of ethylene oxide pendants, and 18 denotes the base coat.

Preferably, the polymer contains a monomer unit comprising one of the following two structures: 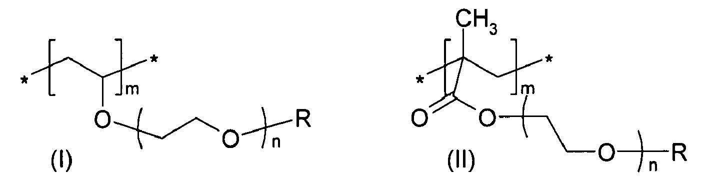 in which R is a hydrogen atom or an alkyl group of 1 to 6 carbon atoms; n is an integer of 2 to 100; and m is an integer of 100 to 5000. Preferably, n is an integer of 2 to 10. The alkyl group suitable for the present invention may be straight, branched, or cyclic. Examples of the suitable alkyl groups include, but do not limit to: methyl, ethyl, propyl, isopropyl, butyl, isobutyl, *tert*-butyl, *n*-pentyl, cyclopropyl, cyclobutyl, and cyclopentyl. Preferably, the alkyl group is methyl.

The polymer may also be a copolymer comprising a monomer unit containing one of the following structures: 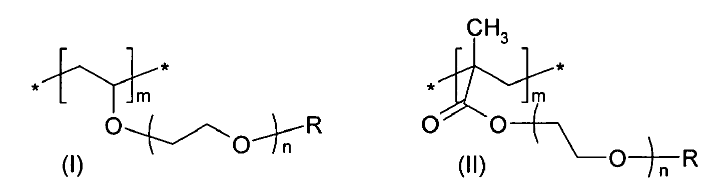 as defined above; and up to 50%mole of one or more methacrylate or acrylate co-monomer units.

The article that may be coated with the amphiphilic coating material may be in any shape, and is preferably a medical device or a component of a medical device. The term "medical device" as used herein denotes a physical item used in medical treatment, which includes both external medical devices and implantable medical devices. The medical devices that may be coated with the amphiphilic coating material include, but are not limited to: catheters, guidewires, drug eluting stents, cochlear implants, retinal implants, gastric bands, neurostimulation devices, muscular stimulation devices, implantable drug delivery devices, intraocular devices, and various other medical devices.

The present amphiphilic coating material may be applied to the surface of an article using conventional coating techniques, such as, for example, spray coating, ultrasonic coating, dip coating, and the like. In a dip coating process, the article is immersed in a bath containing the amphiphilic coating material and then removed. A dwelling time ranging from about 1 minute to about 2 hours may be used depending of the material of construction, complexity of the device, and the desired coating thickness. Next, the article coated with the amphiphilic coating material may be allowed to dry to provide a dry coating. Drying may be accomplished merely by standing at ambient conditions or may be accelerated by heating at mild temperatures, such as about 30°C to about 65°C.

## Claims

1. An amphiphilic coating material for applying on at least a portion of one surface of an article, said amphiphilic coating material comprising a polymer having a hydrophobic backbone derived from a vinyl moiety and hydrophilic pendent chains derived from a polyethylene oxide moiety.

2. The amphiphilic coating material of claim 1, in which the polymer contains a monomer unit comprising one of the following two structures: 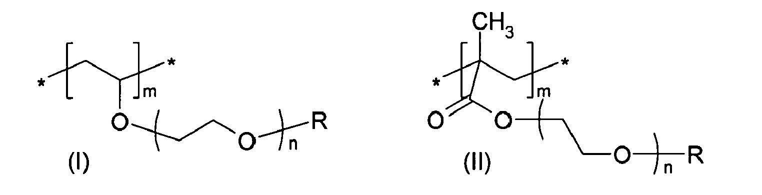 in which R is a hydrogen atom or an alkyl group of 1 to 6 carbon atoms; n is an integer of 2 to 100; and m is an integer of 100 to 5000.

3. The amphiphilic coating material of claim 2, in which n is 2 to 10.

4. The amphiphilic coating material of claim 1, which has a thickness of about 1 to about 500 nm.

5. The amphiphilic coating material of claim 1, in which the polymer has a tunable molecular weight ranging from about 5 kDa to about 500 kDa.

6. The amphiphilic coating material of claim 1, in which the polymer is a copolymer comprising a monomer unit containing one of the following structures: 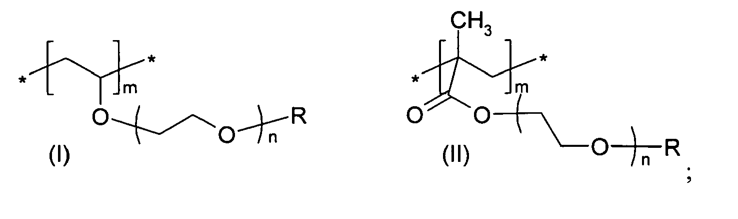 and up to 50% mole of one or more methacrylate or acrylate co-monomer units, in which R is a hydrogen atom or an alkyl group of 1 to 6 carbon atoms; n is an integer of 2 to 100; and m is an integer of 100 to 5000.

7. The amphiphilic coating material of claim 4, in which n is 2 to 10.

8. The amphiphilic coating material of claim 1, in which the polymer is a copolymer comprising the following repeating unit: 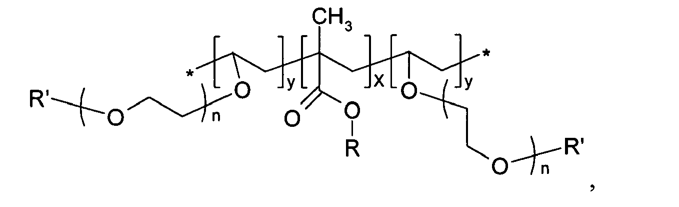 in which R and R' are the same or different, and are independently a hydrogen atom or an alkyl group of 1 to 6 carbons; n is an integer of 2 to 100; and x and y are the same of different, and are independently an integer of 100 to 5000.

9. The amphiphilic coating material of claim 1, in which the polymer is a copolymer comprising the following repeating unit: 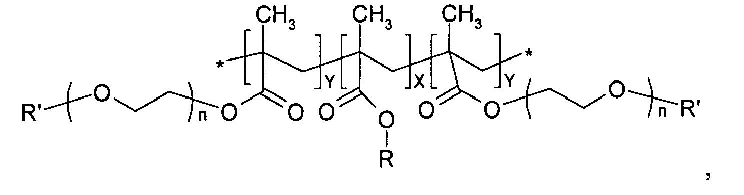 in which R and R' are the same or different, and are independently a hydrogen atom or an alkyl group of 1 to 6 carbons; n is an integer of 2 to 100; and X and Y are the same of different, and are independently an integer of 100 to 5000.

10. An amphiphilic coating material for applying on at least a portion of one surface of an article, said amphiphilic coating material comprising a polymer having a hydrophobic backbone derived from a vinyl moiety and hydrophilic pendent chains derived from a polyethylene oxide moiety, said polymer contains a monomer unit comprising one of the following two structures: 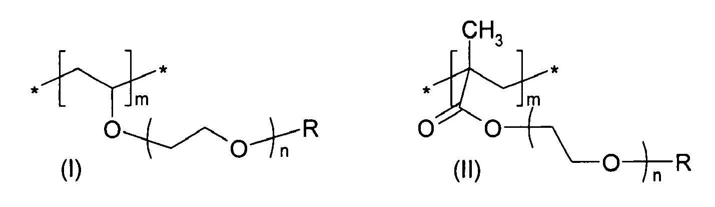 in which R is a hydrogen atom or an alkyl group of 1 to 6 carbon atoms; n is an integer of 2 to 100; and m is an integer of 100 to 5000.

11. An article having an amphiphilic coating thereon, said amphiphilic coating comprising a polymer having a hydrophobic backbone derived from a vinyl moiety and hydrophilic pendent chains derived from a polyethylene oxide moiety.

12. The article of claim 11, in which the polymer contains a monomer unit comprising one of the following two structures: 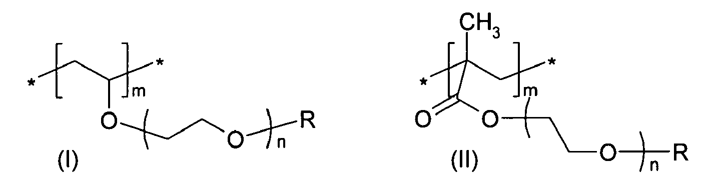 in which R is a hydrogen atom or an alkyl group of 1 to 6 carbon atoms; n is an integer of 2 to 100; and m is an integer of 100 to 5000.

13. The article of claim 12, in which n is an integer of 2 to 10.

14. The article of claim 11, in which the polymer is a copolymer comprising a monomer unit containing one of the following structures: 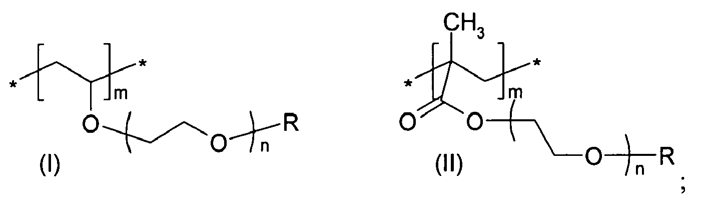 and up to 50% mole of one or more methacrylate or acrylate co-monomer units, in which R is a hydrogen atom or an alkyl group of 1 to 6 carbon atoms; n is an integer of 2 to 100; and m is an integer of 100 to 5000.

15. The article of claim 14, in which n is an integer of 2 to 10.

16. The article of claim 11, in which the amphiphilic coating has a thickness of about 1 to about 500 nm.

17. The article of claim 11, in which the polymer has a tunable molecular weight ranging from about 5 kDa to about 500 kDa.

18. The article of claim 11 is a medical device or a component of a medical device.
